# EUROPEAN PATENT APPLICATION

(11) **EP 1 609 425 A1**
(43) Date of publication of application: **28.12.2005**
(21) Application number: 04714937.2
(22) Date of filing: 26.02.2004
(51) Int. Cl.: A61B 10/00

(54) **SYSTEM FOR TOPICAL NERVE DIAGNOSIS AND NEUROANATOMICAL STUDY**

(30) Priority: 10.03.2003 JP 2003063876; 01.12.2003 JP 2003402116
(71) Applicant: Kamo, Hisaki, Kyoto-shi, Kyoto 610-1102 (JP)
(72) Inventor: KAMO, Hisaki, Nishikyo-ku, Kyoto-shi, Kyoto 610-1102 (JP); OKADA, Naoki, Kyonomichi-cho, Uzumasa, Ukyo-ku,6168181 (JP)
(74) Representative: Gritschneder, Martin
(86) International application number: PCT/JP2004/002327
(87) International publication number: WO 2004/080311

(57) **Abstract**

A system having a whole nerve pathway diagram data recording unit 1; a nerve finding data input unit 2; a responsible nerve pathway data extraction unit 3 by which the data of a nerve pathway responsible for a nerve finding item showing an abnormal finding is extracted from the data of the whole nerve pathway diagram data recording unit based on the data obtained by the nerve finding input unit; a whole nerve pathway indication unit 5 by which the whole nerve pathway is indicated in a display unit 4 based on the data of the whole nerve pathway diagram data recording unit; a responsible nerve pathway indication unit 6 by which the responsible nerve pathway is indicated in the whole nerve pathway diagram based on the data of the whole nerve pathway diagram data recording unit; and a responsible lesion estimation/indication unit 7 by which the location of the responsible lesion in the whole nerve pathway diagram is estimated and indicated in the whole nerve pathway diagram based on the data of the responsible nerve pathway shown in the display unit.

## Description

### FIELD OF THE INVENTION

The present invention relates to a system for topical nerve diagnosis and neuroanatomical study with the use of a computer.

### BACKGROUND ART

In a conventional topical nerve diagnosis, a medical physician presumes responsible nerve pathways which will cause symptoms of abnormality in neural functions such as motor paralysis, perception disorder such as numbness, accommodation disorder in diaphoresis or blood pressure, abnormality in balance or muscle tone, and abnormality in allophasis or tendon reflex from a neural finding with respect to a patient, and decides a responsible lesion based on which the symptom can be explained.

In this case, the medical physician must decide the responsible lesion by the use of own knowledge of neuroanatomy with his (or her) imagination of responsible nerve pathways relating to neural finding items from which such abnormal findings are deduced as well as of mutual physical relations of these responsible nerve pathways. However, knowledge of neuroanatomy required for such diagnostic operation is an enormous amount, so that it is difficult to memorize perfectly the contents of neuroanatomy.

Hence, in a conventional topical nerve diagnosis, a responsible lesion has been decided on the basis of physician's experience and gut feel, so that there was a case where an incorrect diagnosis was made.

Furthermore, medical students are required in a learning of neuroanatomy to read thoroughly books of neuroanatomy, to understand details of nerve pathway diagrams, besides details of nerve pathway cut surface diagrams in respective specified regions of cerebrum, brainstem, spinal cord and the like, and to memorize correctly them. However, nerve pathway diagrams and nerve pathway cut surface diagrams are very complicated, and an amount of information derived therefrom and to be memorized is enormous amount. Accordingly, it was very difficult in general to memorize correctly such information. (For embodiment, see "SHINKEI SHINDANGAKU NYUMON (Principles of Neurologic Diagnosis)" authored by Erwin B. Montogomery, Michael Wall, and Victor W. Henderson; translated by supervision of Shunsaku Hirai; published from Medical Science International; May 1987; and "RINSHOH NO TAMENO SHINKEIKINOU KAIBOHGAKU (Neurological Function Anatomy for Clinic)" authored by Fumio Gotoh and Takahiro Amano; published from Chuhgai Igaku-sha; 1996)

### DISCLOSURE OF THE INVENTION

It is an object of the present invention to provide a system in which a medical physician can make rapidly and correctly a topical nerve diagnosis without relying upon own experience and gut feel.

It is another object of the present invention to provide a system in which medical student can understand easily nerve pathway diagrams and nerve cut surface diagrams in neuroanatomy, and memorize efficiently the contents thereof.

In order to achieve those objects, the present invention provides a topical nerve diagnostic system with the use of a computer, characterized by having a whole nerve pathway diagram data recording unit for storing data of whole nerve pathway diagrams; a nerve finding data input unit for receiving normal finding or abnormal finding data input with respect to respective neural finding items; a responsible nerve pathway data extraction unit for extracting data for drawing a responsible nerve pathway relating to neural finding items being in an abnormal finding from the data stored in the whole nerve pathway diagram data recording unit based on the data received in the nerve finding data input unit; a display unit; a whole nerve pathway indication unit for displaying a whole nerve pathway diagram on the display unit based on the data stored in the whole nerve pathway diagram data recording unit; a responsible nerve pathway indication unit for displaying a responsible nerve pathway in the whole nerve pathway diagram displayed on the display unit by the whole nerve pathway indication unit based on the data extracted by the responsible nerve pathway data extraction unit; and a responsible lesion estimation/indication unit for presuming a position of a responsible lesion in the whole nerve pathway diagram based on the responsible nerve pathway displayed on the display unit by the responsible nerve pathway indication unit.

According to a preferred embodiment of the present invention, the data stored in the whole nerve pathway diagram data recording unit contains data of at least names and positions of respective nerve nuclei in the whole nerve pathway diagram, connection relations in the respective nerve nuclei, and curves or straight lines representing nerve fascicles for connecting the nerve nuclei with each other.

According to another preferred embodiment of the present invention, the responsible nerve pathway data extraction unit is adapted to extract data of relevant names and positions of nerve nuclei in the whole nerve pathway diagram, relevant connection relations in the respective nerve nuclei, and curves or straight lines representing nerve fascicles for connecting the relevant nerve nuclei with each other from the whole nerve pathway diagram data recording unit in every neural finding items exhibiting abnormal findings.

According to a further preferred embodiment of the present invention, the responsible lesion estimation/indication unit is adapted to detect a region where responsible nerve pathways displayed on the display unit intersect with each other and a region where the responsible nerve pathways approach one another in the closest relation, and presume the region detected to be a responsible lesion thereby to display the responsible lesion in the whole nerve pathway diagram on the display unit.

According to a still further preferred embodiment of the present invention, the topical nerve diagnostic system includes a nerve pathway cut surface data recording unit for storing cut surface data in a specified region in the whole nerve pathway diagram; a cut surface display region selection data input unit for receiving selection data input of a specified region in which a cut surface is to be displayed in the whole nerve pathway diagram displayed on the display unit; a second responsible nerve pathway data extraction unit for extracting data for drawing a responsible nerve pathway relating to a neural finding item to be in an abnormal finding onto a cut surface of relevant specified region from the data stored in the nerve pathway cut surface data recording unit based on the data received by the cut surface display region selection data input unit and the data received by the nerve finding data input unit; a nerve pathway cut surface indication unit for extracting relevant cut surface data from the data stored in the nerve pathway cut surface data recording unit based on the data received by the cut surface display region selection data input unit thereby to display the relevant cut surface; a second responsible nerve pathway indication unit for displaying a responsible nerve pathway in the nerve pathway cut surface displayed by the nerve pathway cut surface indication unit based on the data extracted by the second responsible nerve pathway data extraction unit; and a second responsible lesion estimation/indication unit for presuming a position of a responsible lesion in the relevant cut surface based on the responsible nerve pathway displayed on the display unit by the second responsible nerve pathway indication unit thereby to display the responsible lesion presumed in the relevant cut surface.

According to an yet further preferred embodiment of the present invention, the data stored in the nerve pathway cut surface data recording unit contains data of relevant respective names and positions of nerve nuclei in the cut surface, connection relations in relevant respective nerve nuclei, and curves or straight lines representing nerve fascicles for connecting the relevant nerve nuclei with each other in the every cut surfaces.

According to a still further preferred embodiment of the present invention, the second responsible nerve pathway data extraction unit is adapted to extract data of relevant respective names and positions of nerve nuclei in the cut surface, connection relations in relevant respective nerve nuclei, and curves or straight lines representing nerve fascicles for connecting the relevant nerve nuclei with each other from the relevant cut surface data stored in the nerve pathway cut surface data recording unit in every neural finding items to be in abnormal findings.

According to a further preferred embodiment of the present invention, the second responsible lesion estimation/indication unit is adapted to detect a region where responsible nerve pathways displayed on the display unit intersect with each other and a region where the responsible nerve pathways approach one another in the closest relation, and presume the region detected to be a responsible lesion thereby to display the responsible lesion presumed in the cut surface.

According to an yet further preferred embodiment of the present invention, the topical nerve diagnostic system includes a screen page switchover unit for switching over a screen page of the whole nerve pathway diagram in the display unit to a screen page of a cut surface in a specified region of the whole nerve pathway diagram.

According to a still further preferred embodiment of the present invention, the neural finding items include oculomotor restriction, inferior oculomotor restriction, jaw reflex acceleration, impaired facial tactual sensation, impaired facial pain/temperature sensation, corneal areflexia, exterior oculomotor restriction no, upper facial paralysis, lower facial paralysis, impaired taste, lowered pharyngeal reflex/swallowing difficulty, impaired pharyngeal sound dysphemia, lingual muscle paralysis/impaired lingual sound dysphemia, sternocleidomastoid paralysis, impaired upper limb pain/temperature sensation, impaired upper limb deep sensation, upper limb motor paralysis, upper limb tendon reflex acceleration no, impaired trunk pain/temperature sensation, impaired trunk deep sensation, level of impaired trunk deep sensation, impaired lower limb pain/temperature sensation, inferior bathyesthesia disorder, lower limb motor paralysis, lower limb tendon reflex acceleration no, and Babinski reflex.

According to an yet further preferred embodiment of the present invention, the data stored in the whole nerve pathway diagram data recording unit contains at least data of names and positions of respective spinal roots, respective muscles and respective skin areas in the whole nerve pathway diagram, connection relations in the respective spinal roots and the respective muscles, and curves or straight lines representing nerve fascicles for connecting the respective spinal roots with the respective skin as well as data of connection relations in the respective spinal roots and the respective skin areas, and curves or straight lines for connecting the respective spinal roots with the respective skin areas.

According to a further preferred embodiment of the present invention, the responsible nerve pathway data extraction unit is adapted to extract data of relevant names and positions of spinal roots, muscles and skin areas in the whole nerve pathway diagram, relevant connection relations in the respective spinal roots and the respective muscles, and curves or straight lines representing nerve fascicles for connecting the relevant respective spinal roots with the respective skins as well as data of relevant connection relations in the respective spinal roots and the respective skin areas, and curves or straight lines for connecting the relevant respective spinal roots with the respective skin areas from the whole nerve pathway diagram data recording unit in every neural finding items which are to be in abnormal findings.

According to a still further preferred embodiment of the present invention, the responsible lesion estimation/indication unit is adapted to detect a region where responsible nerve pathways displayed on the display unit overlap each other at the highest degree, and presume the region detected to be a responsible lesion thereby to display the responsible lesion presumed in the whole nerve pathway diagram on the display unit.

According to an yet further preferred embodiment of the present invention, the topical nerve diagnostic system includes further a third responsible lesion estimation/indication unit excluding a responsible nerve pathway part corresponding to nerve fascicles for connecting a muscle or skin region in which finding data input comes to be a normal finding with the spinal roots relating thereto from the responsible nerve pathways displayed in the whole nerve pathway diagram on the display unit by means of the responsible lesion estimation/indication unit in the case when the finding data input as to abnormality of respective muscles or respective skin regions relating to the responsible nerve pathways is received by the nerve finding data input unit.

According to a still further preferred embodiment of the present invention, the neural finding items includes perception disorder in respective muscle and skin regions relating to movements of respective articulations.

In order to achieve the above-mentioned objects, the present invention provides a neuroanatomy learning system with the use of a computer, characterized by having a nerve pathway cut surface data recording unit for recording cut surface data in at least one region of cerebrum and mesencephalon, at least one region of pons, at least one region of medulla oblongata, and at least one region of spinal cord, respectively, in a whole pathway diagram; a display unit; a nerve pathway cut surface indication unit for displaying cut surfaces of at least one region of the cerebrum and the mesencephalon, at least one region of the pons, at least one region of the medulla oblongata, at least one region of the medulla oblongata, and at least one region of the spinal cord, respectively, in this order based on the data stored in the nerve pathway cut surface data recording unit; a nerve pathway selection data input unit for receiving selection data input of nerve pathways to be displayed on the display unit; a nerve pathway data extraction unit for extracting data for drawing relevant nerve pathways from the data stored in the nerve pathway cut surface data recording unit based on the data received by the nerve pathway selection data input unit in every nerve pathway cut surfaces; a nerve pathway indication unit for displaying relevant nerve pathways in a nerve pathway cut surface displayed by the nerve pathway cut surface indication unit based on the data extracted by the nerve pathway data extraction unit; a nerve pathway cut surface selection data input unit for receiving selection data input for a nerve pathway cut surface which is intended to individually display among the nerve pathway cut surfaces displayed on the display unit by means of the nerve pathway cut surface indication unit; an individual nerve pathway cut surface data extraction unit for extracting data for drawing a relevant nerve pathway cut surface from the data stored in the nerve pathway cut surface data recording unit based on the data received by the nerve pathway cut surface selection data input unit; an individual nerve pathway cut surface indication unit for displaying a relevant nerve pathway cut surface on the display unit based on the data extracted by the individual nerve pathway cut surface data extraction unit; and a nerve pathway-nerve nucleus name indication unit for displaying a name of a nerve pathway or a nerve nucleus which is selected in the nerve pathway cut surface displayed on the display unit by means of the individual nerve pathway cut surface indication unit.

According to a preferred embodiment of the present invention, the data stored in the nerve pathway cut surface data recording unit contains data of relevant names and positions of nerve nuclei in the cut surfaces, relevant connection relations in the nerve nuclei, and curves or straight lines representing nerve fascicles for connecting relevant nerve nuclei with each other, and names of relevant nerve pathway and positions in the cut surfaces in every cut surfaces.

According to another preferred embodiment of the present invention, at least one region of the mesencephalon consists of the upper part of the mesencephalon and the lower part of the mesencephalon, at least one region of the pons consists of the upper, the middle, and the lower parts of the pons, at least one region of the medulla oblongata consists of the upper part, the upper-middle part, the middle, the middle-lower part, and the lower part of the medulla oblongata, and at least one region of the spinal cord consists of a cervical segment, a thoracic segment, and a lumbar segment.

### BRIEF DESCRIPTION OF THE DRAWINGS

Fig. 1 is a block diagram illustrating a topical nerve diagnostic system according to an embodiment of the present invention.
Fig. 2 is a diagram showing an embodiment of a neural finding data input screen page.
Fig. 3 is a diagram showing an embodiment of a neural finding data input screen page.
Fig. 4 is a diagram showing an embodiment of a neural finding data input screen page.
Fig. 5 is a diagram showing an embodiment of a neural finding data input screen page.
Fig. 6 is a diagram showing an embodiment of a neural finding data input screen page.
Fig. 7 is a diagram showing an embodiment of a neural finding data input screen page.
Fig. 8 is a diagram showing an embodiment of a neural finding data input screen page.
Fig. 9 is a view showing an embodiment of a whole nerve pathway diagram in which responsible nerve pathways are indicated.
Fig. 10 is a view showing an embodiment of a whole nerve pathway diagram in which responsible nerve pathways are indicated.
Fig. 11 is a view showing an embodiment of a whole nerve pathway diagram in which responsible nerve pathways are indicated.
Fig. 12 is a view showing an embodiment of a whole nerve pathway diagram in which responsible nerve pathways are indicated.
Fig. 13 is a view showing an embodiment of a whole nerve pathway diagram in which responsible nerve pathways are indicated.
Fig. 14 is a view showing an embodiment of a whole nerve pathway diagram in which responsible nerve pathways are indicated.
Fig. 15 is a view showing an embodiment of a whole nerve pathway diagram in which responsible nerve pathways are indicated.
Fig. 16 is a view showing an embodiment of a whole nerve pathway diagram in which responsible nerve pathways are indicated.
Fig. 17 is a block diagram illustrating a neuroanatomy learning system according to an embodiment of the present invention.
Fig. 18 is a diagram showing an embodiment of a nerve pathway selection data input screen page in the system shown in Fig. 17.
Fig. 19 is a view showing a part of a series of nerve pathway cut surfaces in which a nerve pathway is indicated.
Fig. 20 is a view showing a part of a series of nerve pathway cut surfaces in which a nerve pathway is indicated.
Fig. 21 is a view showing a part of a series of nerve pathway cut surfaces in which a nerve pathway is indicated.
Fig. 22 is a view showing a part of a series of nerve pathway cut surfaces in which a nerve pathway is indicated.
Fig. 23 is a view showing a part of a series of nerve pathway cut surfaces in which a nerve pathway is indicated.
Fig. 24 is a view showing a part of a series of nerve pathway cut surfaces in which a nerve pathway is indicated.
Fig. 25 is a view showing a part of a series of nerve pathway cut surfaces in which a nerve pathway is indicated.
Fig. 26 is a view showing an embodiment of an individual enlarged view of the nerve pathway cut surfaces.
Fig. 27 is a diagram showing an embodiment of a neural finding data input screen page.
Fig. 28 is a diagram showing an embodiment of a neural finding data input screen page.
Fig. 29 is a view showing segments of a skin area.
Fig. 30 is a view showing an embodiment of a whole nerve pathway diagram in which responsible nerve pathways are indicated.

### BEST MODE FOR CARRYING OUT THE INVENTION

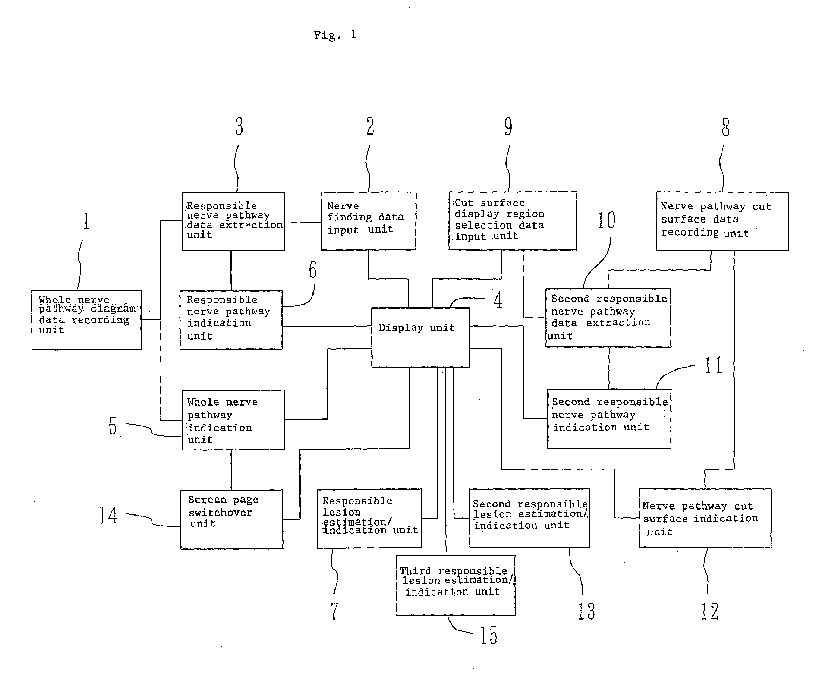

In the following, preferred embodiments of the present invention will be described by referring to the accompanying drawings. Fig. 1 is a block diagram illustrating a topical nerve diagnostic system according to an embodiment of the present invention. The topical nerve diagnostic system is that utilizes a computer and operates independently in two modes of a central topical nerve diagnosis mode and a peripheral topical nerve diagnosis mode.

Referring to Fig. 1, the topical nerve diagnostic system involves a whole nerve pathway diagram data recording unit 1 for storing data of the whole nerve pathway diagram. The data stored in the whole nerve pathway diagram data recording unit 1 contains at least data of respective names and positions of nerve nuclei in the whole nerve pathway diagram, connection relations in the respective nerve nuclei, and curves or straight lines representing nerve fascicles for connecting nerve nuclei with each other for the purpose of diagnosis of central neural system, while at least data of names and positions in the whole nerve pathway diagram of respective spinal roots, respective muscles and respective skin areas, connection relations between the respective spinal roots and the respective muscles, and curves or straight lines representing nerve fascicles for connecting spinal roots with muscles, respectively, as well as data of curves and straight lines representing nerve fascicles for connecting the respective spinal roots with the respective skin areas for the purpose of diagnosis of peripheral neural system.

In the present embodiment, names and positions in the whole nerve pathway diagram of the respective skin areas are previously determined from the results obtained by superposing a spinal rooting sense dominant diagram to a peripheral neural sense dominant diagram as shown in Fig. 29.

The system of the present invention is further provided with a nerve finding data input unit 2 for receiving input for data of normal finding or abnormal finding with respect to respective neural finding items, a responsible nerve pathway data extraction unit 3 for extracting data for drawing a responsible nerve pathway relating to a neural finding item which becomes an abnormal finding from the data stored in the whole nerve pathway diagram data recording unit 1 on the basis of the data received in the nerve finding data input unit 2, and a display unit 4.

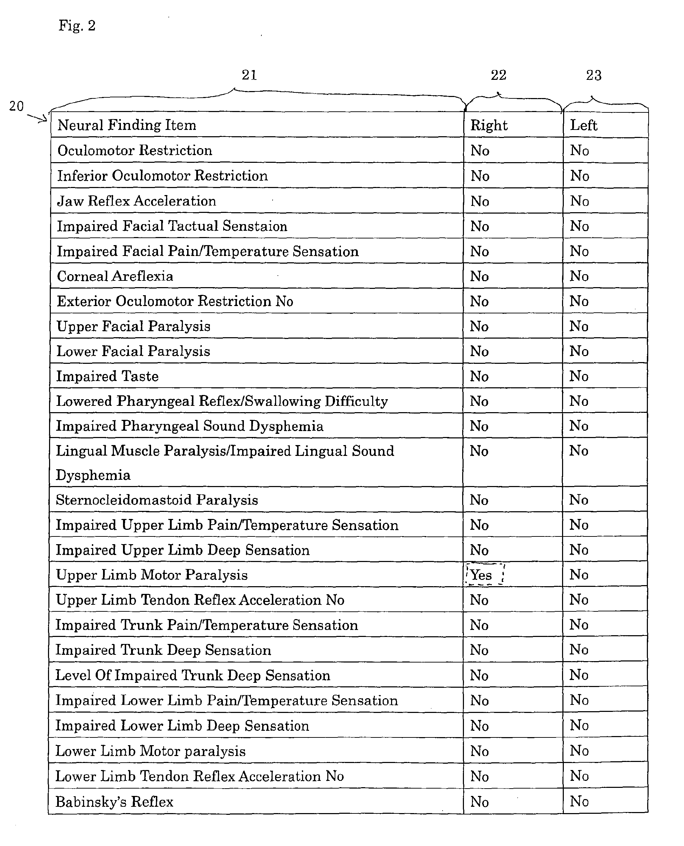

The nerve finding data input unit 2 displays the data input screen page on the display unit 4 as shown in Fig. 2 in a central topical nerve diagnosis mode. Referring to Fig. 2, the data input screen page has a form in a table 20 containing a neural finding item indication column 21 wherein respective neural finding items are vertically laid out, and finding data input columns 22 and 23 for each inputting differentiations in either a normal finding or an abnormal finding corresponding to a specific neural finding item indicated in the neural finding item indication column 21 ("no" is input in case of a normal finding, while "yes" is input in case of an abnormal finding in the present embodiment). The finding data input columns 22 and 23 consist of the column 22 to which finding data relating to the left side of a human body are input and the column 23 to which finding data relating to the right side of a human body are input.

In this case, the neural finding items include oculomotor restriction, inferior oculomotor restriction, jaw reflex acceleration, impaired facial tactual sensation, impaired facial pain/temperature sensation, corneal areflexia, exterior oculomotor restriction no, upper facial paralysis, lower facial paralysis, impaired taste, lowered pharyngeal reflex/swallowing difficulty, impaired pharyngeal sound dysphemia, lingual muscle paralysis/impaired lingual sound dysphemia, sternocleidomastoid paralysis, impaired upper limb pain/temperature sensation, impaired upper limb deep sensation, upper limb motor paralysis, upper limb tendon reflex acceleration no, impaired trunk pain/temperature sensation, impaired trunk deep sensation, level of impaired trunk deep sensation, impaired lower limb pain/temperature sensation, impaired lower limb deep sensation, lower limb motor paralysis, lower limb tendon reflex acceleration no, and Babinski reflex. However, neural finding items are not limited to those specified in the present embodiment, but the other neural finding items may be added.

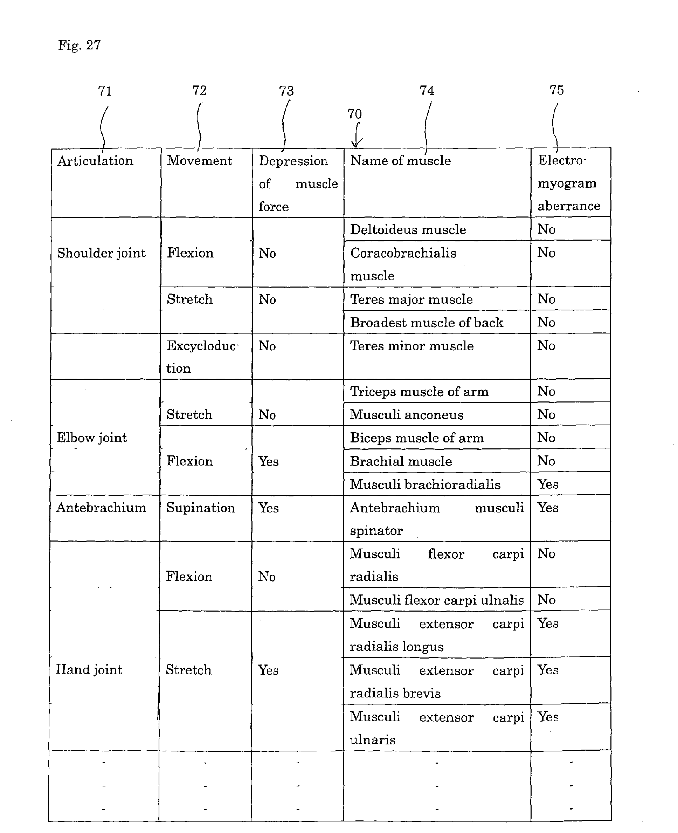

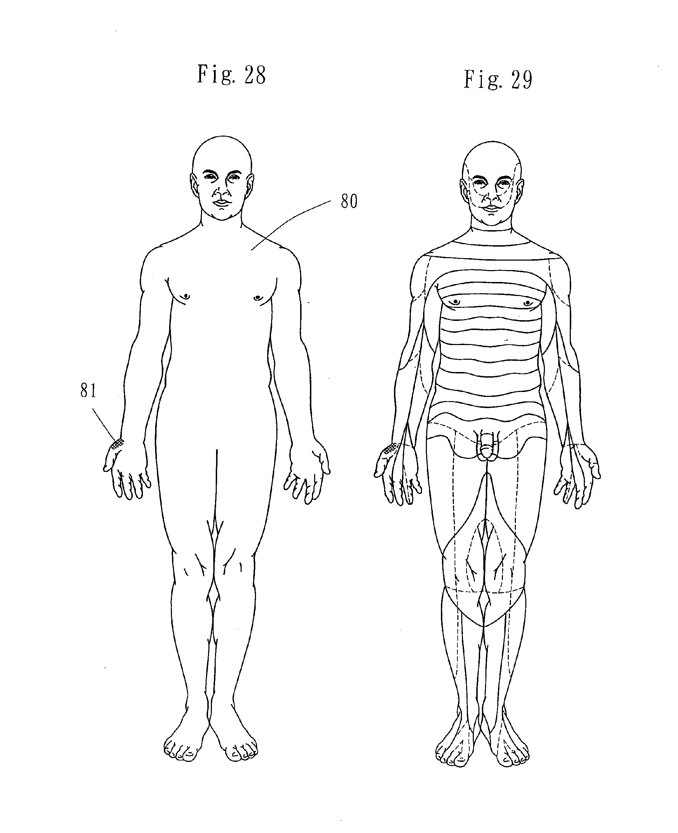

Furthermore, the nerve finding data input unit 2 displays data input screen pages as shown in Figs. 27 and 28 on the display unit 4 in the peripheral topical nerve diagnosis mode. In this case, the data input screen page shown in Fig. 27 is that for inputting neural finding data relating to motor nerve system, while the data input screen page shown in Fig. 28 is that for inputting neural finding data relating to sensory nerve system.

Referring to Fig. 27, the data input screen page has a form in a table 70 containing a glenoid name indication column 71 wherein respective glenoid names are vertically laid out, finding data input columns 72 and 73 for each inputting differentiations in either a normal finding or an abnormal finding with respect to respective articular movements (such as bending and stretching), more specifically, presence of decrease in muscle force in bending, stretching and the like movements as a result of empty-handed muscle force test, muscle name indication column 74 in which muscle names relating to movements of respective articulations are indicated, and a finding data input column 75 for inputting finding data with respect to abnormality in respective muscles. Although finding data with respect to abnormality in respective muscles can be acquired by means of a variety of well-known manners, they are obtained by, for embodiment, checking abnormality in electromyograms of respective muscles in the present embodiment. Accordingly, the finding data input column 75 is in the form of an electromyogram finding data input column 75 in the table 70 of Fig. 27.

Next, referring to Fig. 28, the data input screen page is in the form of a human body plan view 80. The human body plan view 80 is united into a predetermined number of regions (skin areas) as shown in Fig. 29. In this respect, it is arranged in such that when a region where a sensorial disorder arises is pointed out by means of, for embodiment, an appropriate pointing device such as a mouse on the human body plan view 80, data of a name corresponding to the skin area and a position in the whole nerve pathway diagram are input, so that finding data as to the sensorial disorder (nerve finding items) in respective skin areas are input.

The responsible nerve pathway data extraction unit 3 extracts data of relevant names and positions of nerve nuclei in the whole nerve pathway diagram, relevant connection relations in the respective nerve nuclei, and data of curves or straight lines representing nerve fascicles for connecting relevant nerve nuclei with each other from the whole nerve pathway diagram data recording unit 1 in every neural finding items which exhibit abnormal findings in a central topical nerve diagnosis mode. Furthermore, the responsible nerve pathway data extraction unit 3 extracts data of relevant names and positions of spinal roots, muscles and skin areas in the whole nerve pathway diagram, relevant connection relations in the respective spinal roots and the respective muscles, and data of curves or straight lines representing nerve fascicles for connecting the relevant respective spinal roots with the respective skins as well as data of relevant connection relations in the respective spinal roots and the respective skin areas, and curves or straight lines for connecting the relevant respective spinal roots with the respective skin areas from the whole nerve pathway diagram data recording unit 1 in every neural finding items which exhibit abnormal findings in a peripheral topical nerve diagnosis mode.

Furthermore, the responsible nerve pathway data extraction unit 3 extracts data of relevant names and positions of spinal roots, muscles and skin areas in the whole nerve pathway diagram, relevant connection relations in the respective spinal roots and the respective muscles, and data of curves or straight lines representing nerve fascicles for connecting the relevant respective spinal roots with the respective skins as well as data of relevant connection relations in the respective spinal roots and the respective skin areas, and curves or straight lines for connecting the relevant respective spinal roots with the respective skin areas from the whole nerve pathway diagram data recording unit 1 in every neural finding items which exhibit abnormal findings in a peripheral topical nerve diagnosis mode.

The system of the present invention is further provided with a whole nerve pathway indication unit 5 for displaying the whole nerve pathway diagram on the display unit 4 based on the data stored in the whole nerve pathway diagram data recording unit 1, and a responsible nerve pathway indication unit 6 for displaying responsible nerve pathways in the whole nerve pathway diagram displayed in the display unit 4 by the whole nerve pathway indication unit 5 based on the data extracted by the responsible nerve pathway data extraction unit 3.

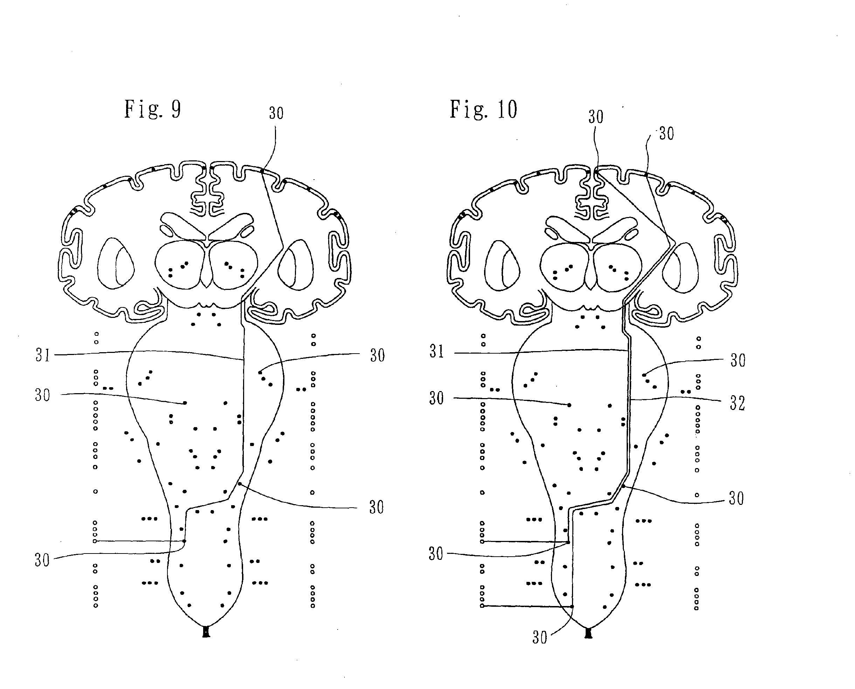

Fig. 9 shows the whole nerve pathway diagram displayed in the display unit 4, and an embodiment of responsible nerve pathways in the central topical nerve diagnosis mode. In Fig. 9, only an outline of the whole nerve pathway diagram, nerve nuclei, and responsible nerve pathways are shown for the clarity. In Fig. 9, reference numeral 30 designates a nerve nucleus, and reference numeral 31 designates a responsible nerve pathway displayed by the responsible nerve pathway indication unit 6.

It is preferred that the whole nerve pathway diagram and the responsible nerve pathway as well as different responsible nerve pathways themselves are displayed with different colors one another on the display unit 4. As a result, it becomes possible to clearly recognize visually the whole nerve pathway diagram and respective responsible nerve pathways.

The system of the present invention comprises further a responsible lesion estimation/indication unit 7 which presumes a position of a responsible lesion in the whole nerve pathway diagram on the basis of the responsible nerve pathway displayed on the display unit 4 by means of the responsible nerve pathway indication unit 6, and displays the presumed responsible lesion in the whole nerve pathway diagram.

The responsible lesion estimation/indication unit 7 is adapted to detect a region where responsible nerve pathways displayed on the display unit 4 intersect with each other and a region where responsible nerve pathways approach one another with the closest relation, and presume the region detected to be a responsible lesion thereby to display the lesion in the whole nerve pathway diagram of the display unit 4 in the central topical nerve diagnosis mode. Moreover, the responsible lesion estimation/indication unit 7 is adapted to detect a region where responsible nerve pathways displayed on the display unit 4 overlap with each other at the highest degree, and presume the region detected to be a responsible lesion thereby to display the lesion in the whole nerve pathway diagram of the display unit 4 in the peripheral topical nerve diagnosis mode. It is preferred that such responsible lesion is displayed on the display unit 4 with a different color from those of the whole nerve pathway diagram and the responsible nerve pathways.

Furthermore, the system of the present invention includes a third responsible lesion estimation/indication unit 15 which excludes a responsible nerve pathway part corresponding to nerve fascicles for connecting a muscle a finding data (electromyogram data) of which is to be a normal finding with the relevant spinal roots, the finding data being input from the data input screen page shown in Fig. 27, from the responsible nerve pathways displayed in the whole nerve pathway diagram of the display unit 4 by means of the responsible lesion estimation/indication unit 7 in the case when data of a normal finding or an abnormal finding is received by the nerve finding data input unit 2 with respect to an electromyogram of respective muscles relating to the responsible nerve pathways in a peripheral topical nerve diagnosis mode.

The system of the present invention includes further a nerve pathway cut surface data recording unit 8 for storing data of segments in a specified region in the whole nerve pathway diagram. The data stored in the nerve pathway cut surface data recording unit 8 contains data of respective relevant names and positions of nerve nuclei in the cut surfaces, relevant connection relations in the respective nerve nuclei, and data of curves or straight lines representing nerve fascicles for connecting relevant nerve nuclei with each other in every cut surfaces.

Moreover, the system of the present invention is provided with a cut surface display region selection data input unit 9 for receiving selection data input in a specified region which shall display a cut surface in the whole nerve pathway diagram displayed on the display unit 4, and a second responsible nerve pathway data extraction unit 10 for extracting data for drawing responsible nerve pathways relating to neural finding items which are to be abnormal findings on a cut surface in a relevant specified region from the data stored in the nerve pathway cut surface data recording unit 8 on the basis of the data received by the cut surface display region selection data input unit 9 and the data received by the finding data input unit.

The second responsible nerve pathway data extraction unit 10 is adapted to extract data of relevant names and positions of nerve nuclei in the cut surfaces, relevant connection relations in the respective nerve nuclei, and data of curves or straight lines representing nerve fascicles for connecting relevant nerve nuclei with each other from the data of the relevant cut surfaces stored in the nerve pathway cut surface data recording unit 8 in every neural finding items which exhibit abnormal findings.

The system of the present invention is further provided with a nerve pathway cut surface indication unit 12 which extracts relevant data of cut surfaces from the data stored in the nerve pathway cut surface data recording unit 8 based on the data received by the cut surface display region selection data input unit 9 to display relevant cut surfaces, and a second responsible nerve pathway indication unit 11 which displays responsible nerve pathways in a nerve pathway cut surface displayed by the nerve pathway cut surface indication unit 12 based on the data extracted by the second responsible nerve pathway data extraction unit 10.

In this case, it is preferred that nerve pathway cut surfaces and responsible nerve pathways as well as different responsible nerve pathways one another are displayed with different colors on the display unit 4, whereby it becomes possible to clearly recognize visually the whole nerve pathway diagram and respective responsible nerve pathways.

The system of the present invention includes a second responsible lesion estimation/indication unit 13 for presuming a position of a responsible lesion in relevant cut surfaces on the basis of a responsible nerve pathway displayed on the display unit 4 by means of the second responsible nerve pathway indication unit 11 thereby to display the responsible lesion presumed in the cut surface.

The second responsible lesion estimation/indication unit 13 is adapted to detect a region where responsible nerve pathways displayed on the display unit 4 intersect with each other and a region where responsible nerve pathways approach one another in the closest relation, and presume the region detected to be a responsible lesion thereby to display the responsible lesion in the cut surface. In this case, it is preferred that such responsible lesion is displayed on the display unit 4 with a different color from those of the nerve pathway cut surface and the responsible nerve pathways.

Moreover, the system of the present invention is provided with a screen page switchover unit 14 for switching over a screen page of a whole nerve pathway diagram on the display unit 4 to a screen page of a cut surface in a specified region of the whole nerve pathway diagram in a central topical nerve diagnosis mode.

In the following, operations of the topical nerve diagnostic system will be described. First, a case where the system according to the present invention is operated in a central topical nerve diagnosis mode will be described.

For instance, it is supposed that there are observed (1) right side paralysis (paralysis of right limbs), (2) left peripheral facial paralysis, (3) abduction disorder of left eye (paralysis of abducent nerve), (4) sthenia of tendon reflex in right limbs, and (5) Babinski reflex as a result of neural finding with respect to a patient.

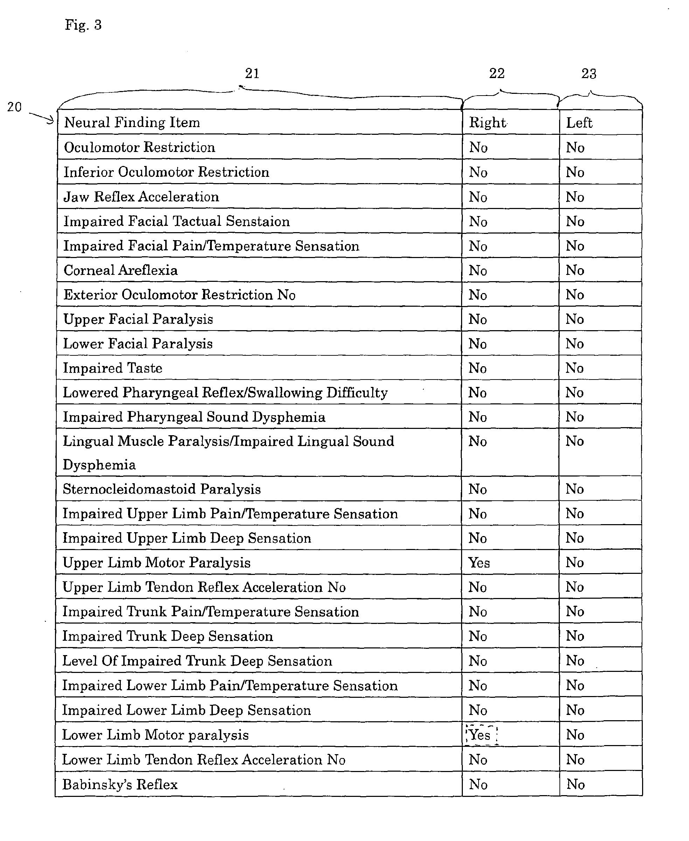

In this case, "yes" is input to the right side of human body finding input column in a neural finding item "upper limb motor paralysis" as shown in Fig. 2 in a data input screen page displayed on the display unit 4 of the system according to the present invention. At this moment, a screen page shown in Fig. 9 is displayed on the display unit 4, and the responsible nerve pathway 31 relating to an abnormal finding of "upper limb motor paralysis" on the right side of human body is displayed in the whole nerve pathway diagram of the display unit 4. Next, "yes" is input to the right side of human body finding input column in a neural finding item "lower limb motor paralysis" as shown in Fig. 3 in a data input screen page. At this moment, a screen page shown in Fig. 10 is displayed on the display unit 4, and the responsible nerve pathway 32 relating to an abnormal finding of "lower limb motor paralysis" on the right side of human body is additionally displayed in the whole nerve pathway diagram.

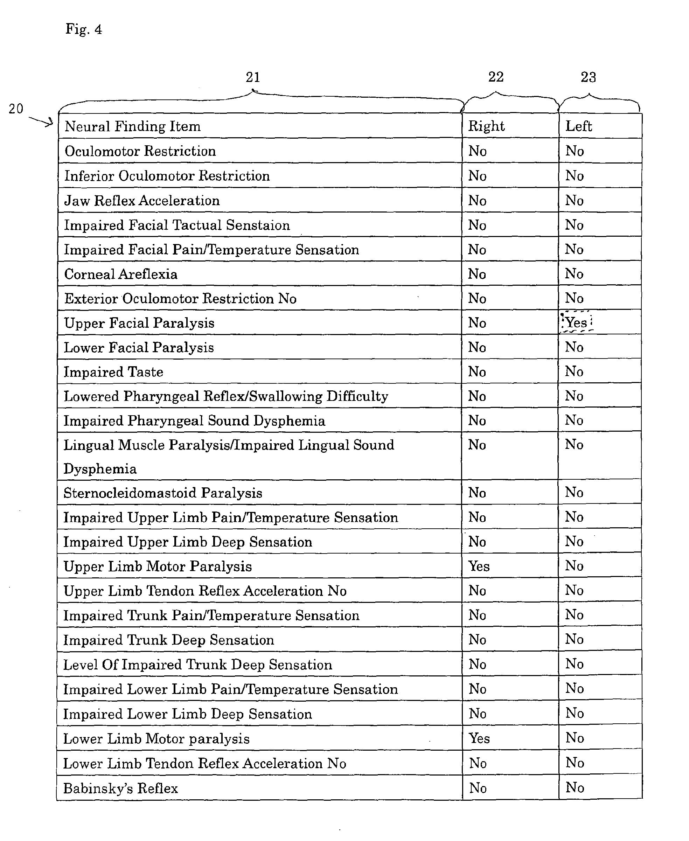

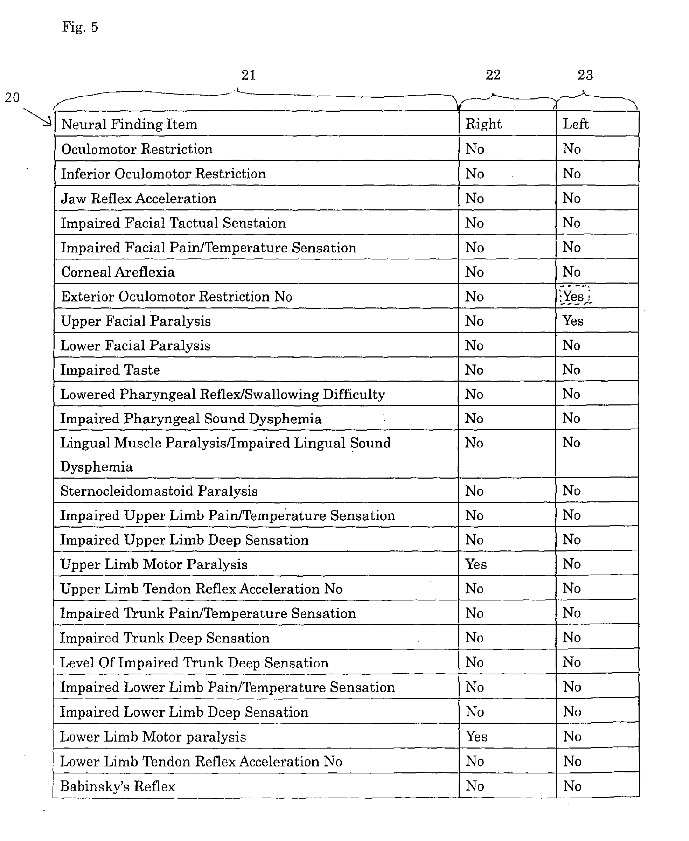

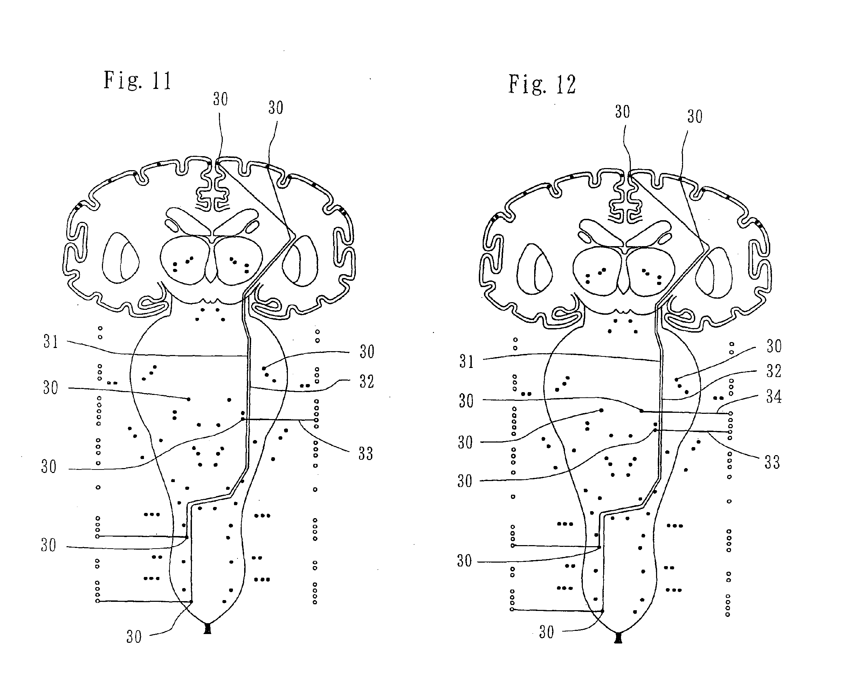

Then, "yes" is input to the left side of human body finding input column in a neural finding item "upper facial paralysis" as shown in Fig. 4 in a data input screen page. At this moment, a screen page shown in Fig. 11 is displayed on the display unit 4, and the responsible nerve pathway 33 relating to an abnormal finding of "upper facial paralysis" on the left side of human body is additionally displayed in the whole nerve pathway diagram. Next, "yes" is input to the left side of human body finding input column in a neural finding item "exterior oculomotor restriction no" as shown in Fig. 5 in a data input screen page. At this moment, a screen page shown in Fig. 12 is displayed on the display unit 4, and the responsible nerve pathway 34 relating to an abnormal finding of "exterior oculomotor restriction no" on the left side of human body is additionally displayed in the whole nerve pathway diagram.

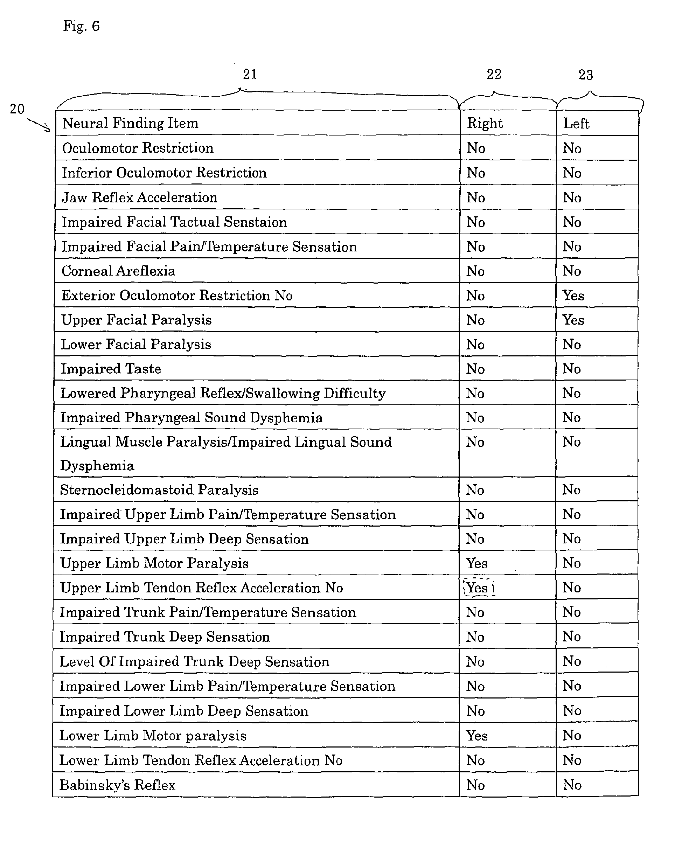

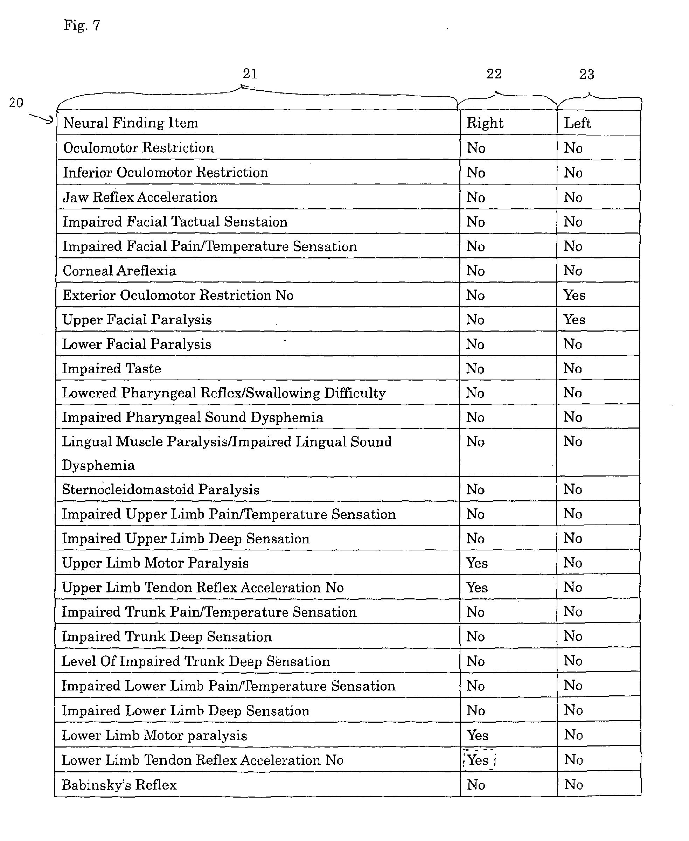

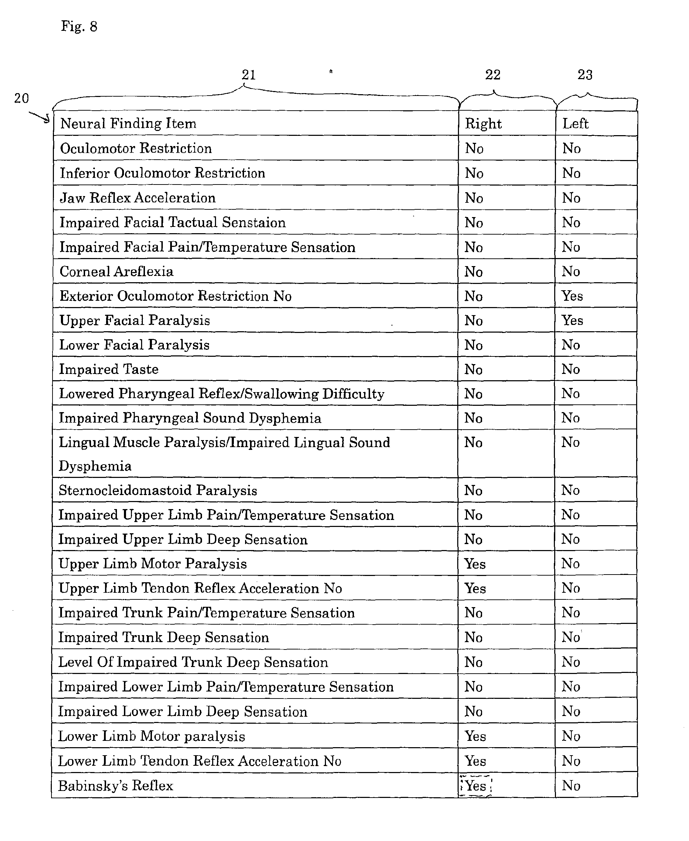

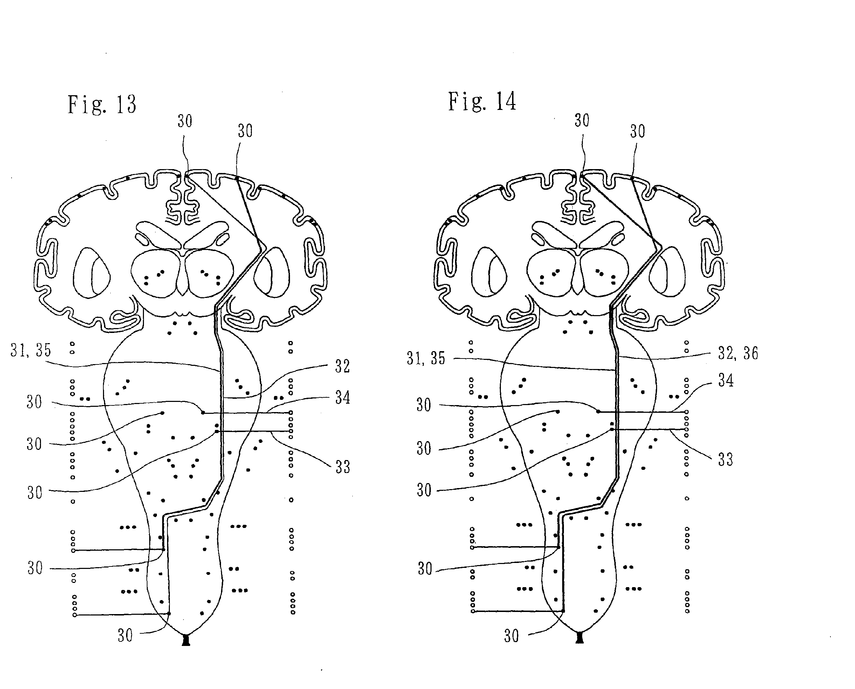

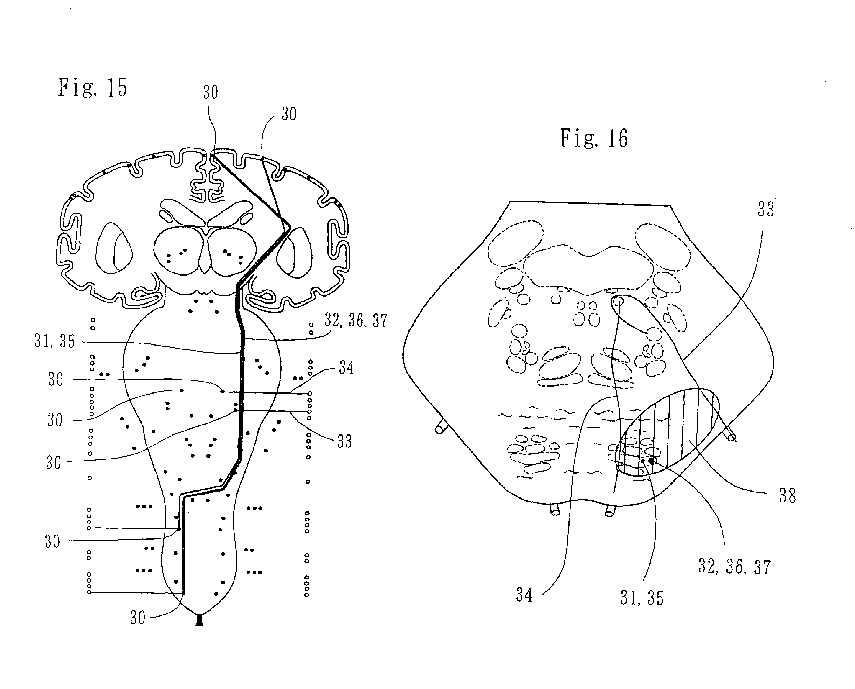

Thereafter, "yes" is input to the right side of human body finding input column in a neural finding item "upper limb tendon reflex acceleration no" as shown in Fig. 6 in a data input screen page. At this moment, a screen page shown in Fig. 13 is displayed on the display unit 4, and the responsible nerve pathway 35 relating to an abnormal finding of "upper limb tendon reflex acceleration no" on the right side of human body is additionally displayed in the whole nerve pathway diagram (a part of the responsible nerve pathway 31 is thickened in this case). Then, "yes" is input to the right side of human body finding input column in a neural finding item "lower limb tendon reflex acceleration no" as shown in Fig. 7 in a data input screen page. At this moment, a screen page shown in Fig. 14 is displayed on the display unit 4, and the responsible nerve pathway 36 relating to an abnormal finding of "lower limb tendon reflex acceleration no" on the right side of human body is additionally displayed in the whole nerve pathway diagram (a part of the responsible nerve pathway 32 is thickened in this case). Moreover, "yes" is input to the right side of human body finding input column in a neural finding item "Babinski reflex" as shown in Fig. 8 in a data input screen page. At this moment, a screen page shown in Fig. 15 is displayed on the display unit 4, and the responsible nerve pathway 37 relating to an abnormal finding of "Babinski reflex" on the right side of human body is additionally displayed in the whole nerve pathway diagram (a part of the responsible nerve pathway 36 is thickened in this case).

When inputs for neural finding items are completed with respect to a patient, a responsible lesion is presumed on the basis of the responsible nerve pathways 32 to 37 (see Fig. 15) displayed on the display unit 4, and the result is displayed in the whole nerve pathway diagram. In case of the present embodiment, a substantially central area at the lower part of a left brainstem is detected as a region where the responsible nerve pathways 32 to 37 are in the closest positions one another, so that the region is presumed to be a responsible lesion, and it is displayed in the whole nerve pathway diagram. This is a brainstem abdominal side syndrome (Millard-Gubler syndrome) observed frequently as one of cerebral infarctions in brainstem. This affection is an important brainstem infarction syndrome as a so-called "alternating hemiplegia" in view of neurology, which exhibits such a situation where a side of paralysis in superior and inferior limbs is reverse with respect to that of facial paralysis, so that it is requested to understand complicated nerve pathways from the viewpoint of diagnosis.

In the whole nerve pathway diagram shown in Fig. 15, when a button for displaying a cut surface in a brainstem area is clicked, the cut surface of the brainstem area is displayed on the display unit 4, and a responsible lesion 38 is also shown in the cut surface as shown in Fig. 16.

Thus, according to a topical nerve diagnostic system of the present invention, when only normal or abnormal finding data is input in every neural finding items, a relevant responsible nerve pathway is displayed automatically together with a whole nerve pathway diagram, whereby a responsible lesion is automatically displayed with respect to a neural disease of a patient. Accordingly, a medical physician can give rapidly and correctly a diagnosis, which is not, made in accordance with physician's experience and gut feeling as in a conventional diagnosis.

Although it is a rare case, such a case where a presumed responsible lesion is displayed on the display unit 4 as a lump containing not only an actual responsible nerve pathway, but also a normal nerve pathway, which is not a responsible nerve pathway, is supposed. In this case, all the neural findings corresponding to such responsible nerve pathways passing through the presumption lesion are made to be displayed on the display unit 4 based on the data stored in the whole nerve pathway diagram data recording unit 1, the respective abnormal findings displayed are reviewed, required additional inspections are implemented, so that operations for eliminating nerve pathways in which no abnormal finding is observed in reality are repeatedly carried out to narrow down a responsible lesion presumed, whereby it becomes possible to presume the responsible lesion at a higher precision.

In the following, the system according to the present invention will be described with respect to the case where the system is operated in a peripheral topical nerve diagnosis mode. For instance, it is supposed that a decrease in muscle force is observed with respect to bending of an ancon articulation and stretching of a hand articulation as a result of empty-handed muscle force test as a neural finding of a motor nerve system as to a patient, and a perception disorder is observed in the skin area 81 shown in Fig. 29 as a neural finding of a sensory nerve system.

In the above case, "yes" is input to a finding input column of "decrease in muscle force" of a finding item "bending movement" as to "ancon articulation" and at the same time, "yes" is input to a finding input column of "decrease in muscle force" of a finding item "stretching movement" as to "hand articulation". Furthermore, the skin area 81 is specified by a pointing device in the data input screen page in Fig. 28, and a name and a position are input in the skin area.

At this moment, a screen page shown in Fig. 29 is displayed on the display unit 4, and a responsible nerve pathway 90 concerning abnormal finding of a bending movement in an ancon articulation and abnormal finding of perception disorder in the skin area 81 are displayed in the whole nerve pathway diagram of the display unit 4. In Fig. 29, reference numerals a to q designate muscles or skin areas relating to questioned abnormal findings wherein a represents deltoideus muscle, b represents teres minor muscle, c represents long head of triceps muscle of the arm, d represents lateral head of triceps muscle of the arm, e represents musculi anconeus, f represents musculi brachioradialis, g represents musculi extensor carpi radialis longus, h represents musculi extensor carpi radialis brevis, i represents musculi spinator, j represents musculi extensor carpi ulnaris, k represents musculi extensor digitorum, 1 represents musculi extensor digiti minimi, m represents musculi abductor pollicis longus, n represents musculi extensor pollicis longus, o represents musculi extensor pollicis brevis, p represents musculi extensor indicis, and q represents radial nerve skin perception branch. Furthermore, A and B designate spinal roots relating to questioned abnormal findings, respectively, and S designates spinal cord. In the circumstances, the muscles and skin areas a to q are connected with relevant spinal roots A and B through nerve fascicles, respectively.

When neural finding item input is completed with respect to a patient, a responsible lesion is presumed on the basis of the responsible nerve pathway 90 displayed on the display unit 4, whereby the responsible lesion is displayed in the whole nerve pathway diagram. In case of the present embodiment, an area extending from a skin area 91 to the musculi brachioradialis is detected as an area where responsible nerve pathways overlap the most frequently with each other, so that it is presumed to be a responsible lesion, and it is displayed in the whole nerve pathway diagram.

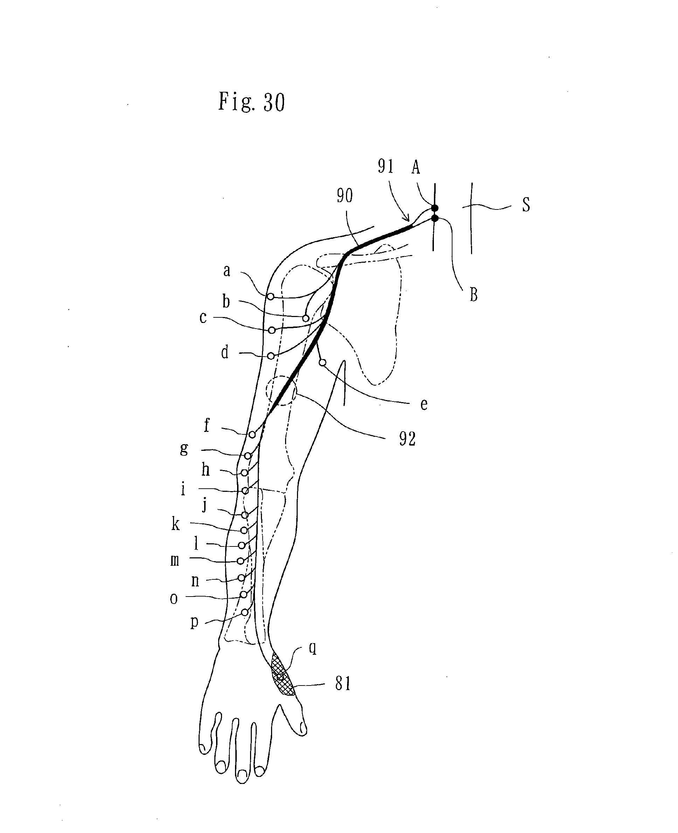

In order to presume more precisely the responsible lesion, a finding due to an electromyogram is added. In this case, "yes" or "no" as to abnormality in the electromyogram is input to the electromyogram finding data input column 72 in a data input screen page (see Fig. 27) displayed on the display unit 4. Now, for instance, it is supposed with reference to Fig. 30 that normal findings in the electromyogram are observed in an extent from the reference character a (deltoideus muscle) to the reference character e (musculi anconeus) in a direction from the spinal cord S to a peripheral region, but an abnormal finding in the electromyogram is observed at the reference character f (musculi brachioradialis). In this case, in Fig. 30, a responsible nerve pathway part for connecting spinal roots A and B relating to the a (deltoideus muscle) to the e (musculi anconeus) is removed from the responsible nerve pathway 90 displayed in the whole nerve pathway diagram. Then, an area 92 is detected in the remaining part as a region 92 where responsible nerve pathways overlap the most frequently with each other, so that it is presumed to be a responsible lesion, and it is displayed in the whole nerve pathway diagram.

The above-mentioned embodiment relates to a system wherein a responsible nerve pathway is displayed by utilizing a computer in a whole nerve pathway diagram or a nerve pathway cut surface from a neural finding with respect to a patient. In this respect, a responsible nerve pathway is the same as an anatomical functional pathway of a whole nerve pathway containing motor and perception pathways, after all. Accordingly, the present invention is also applicable for learning of neuroanatomy.

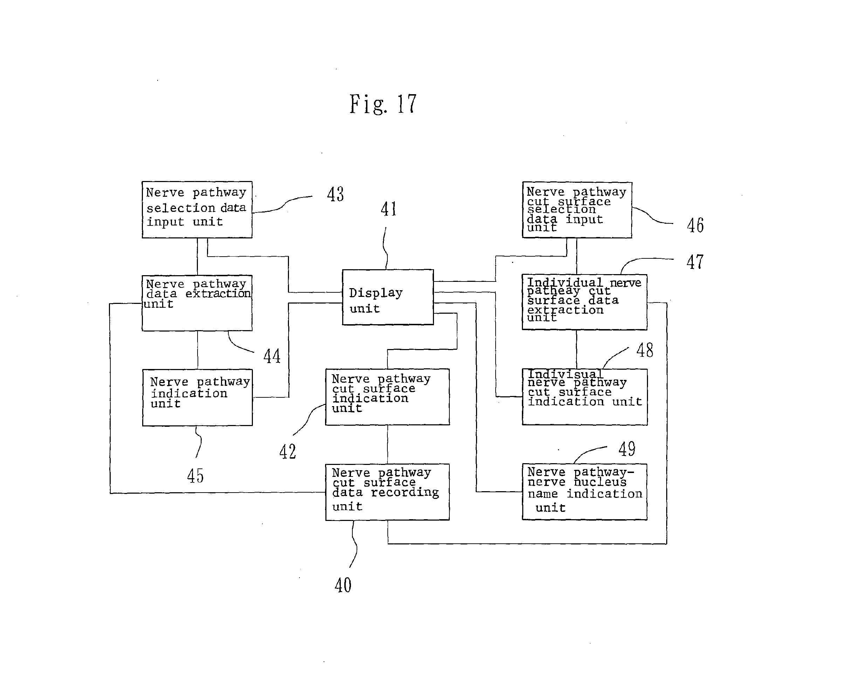

Fig. 17 is a block diagram illustrating a neuroanatomy learning system according an embodiment of the present invention. The neuroanatomy learning system of the present invention utilizes a computer and, as shown in Fig. 17, which is provided with a nerve pathway cut surface data recording unit 40 for recording data of cut surfaces in at least one region of cerebrum and mesencephalon, at least one region of pons, at least one region of medulla oblongata, and at least one region of spinal cord, respectively, in a whole pathway diagram, a display unit 41, and a nerve pathway cut surface indication unit 42 for displaying cut surfaces of at least one region of the cerebrum and the mesencephalon, at least one region of the pons, at least one region of the medulla oblongata, at least one region of the medulla oblongata, and at least one region of the spinal cord, respectively, in this order based on the data stored in the nerve pathway cut surface data recording unit 40.

In the present embodiment, at least one region of the mesencephalon consists of the upper part of the mesencephalon and the lower part of the mesencephalon, at least one region of the pons consists of the upper, the middle, and the lower parts of the pons, at least one region of the medulla oblongata consists of the upper part, the upper-middle part, the middle, the middle-lower part, and the lower part of the medulla oblongata, and at least one region of the spinal cord consists of a cervical segment, a thoracic segment, and a lumbar segment.

The data stored in the nerve pathway cut surface data recording unit 40 contains data of relevant names and positions of nerve nuclei in the cut surfaces, relevant connection relations in the nerve nuclei, and data of curves or straight lines representing nerve fascicles for connecting relevant nerve nuclei with each other, and names of relevant nerve pathway and positions in the cut surfaces in every cut surfaces.

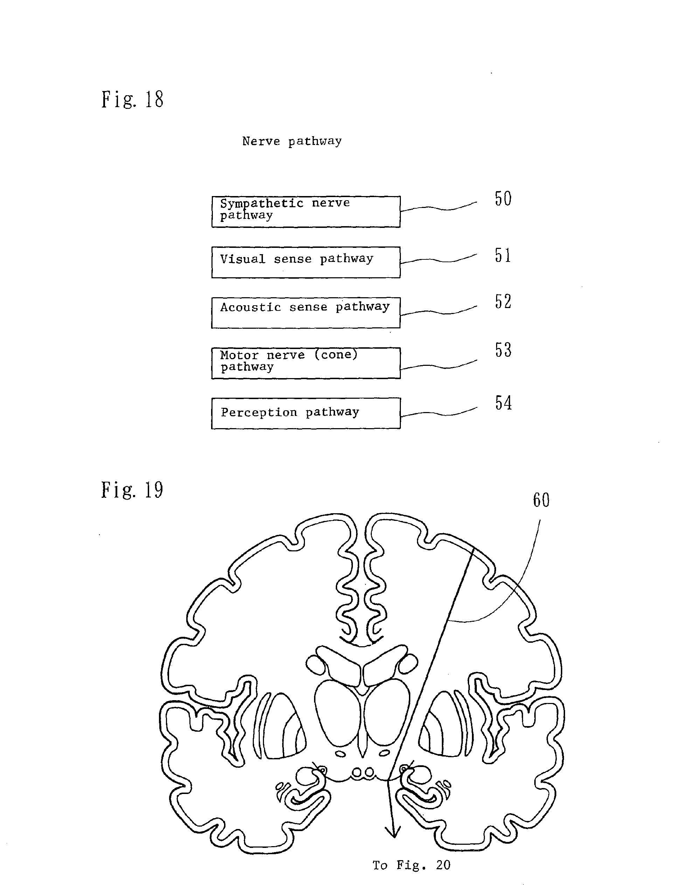

The system of the present invention is further provided with a nerve pathway selection data input unit 43 for receiving selection data of nerve pathways to be displayed on the display unit. Fig. 18 illustrates a nerve pathway selection data input screen page displayed on the display unit 41 by means of the nerve pathway selection data input unit 43. As shown in Fig. 18, the nerve pathway selection data input screen page includes a sympathetic nerve pathway display button 50, a visual sense pathway display button 51, an acoustic sense pathway display button 52, a motor nerve (cone) pathway display button 53, and a perception pathway display button 54 wherein when a desired button is pressed by means of a pointing device such as a mouse, a nerve pathway to be displayed is selected.

The system of the present invention is further provided with a nerve pathway data extraction unit 44 for extracting data for drawing relevant nerve pathway from the data stored in the nerve pathway cut surface data recording unit 40 based on the data received by the nerve pathway selection data input unit 43 in every nerve pathway cut surfaces, and a nerve pathway indication unit 45 for displaying relevant nerve pathways in a nerve pathway cut surface displayed by the nerve pathway cut surface indication unit 42 based on the data extracted by the nerve pathway data extraction unit 44.

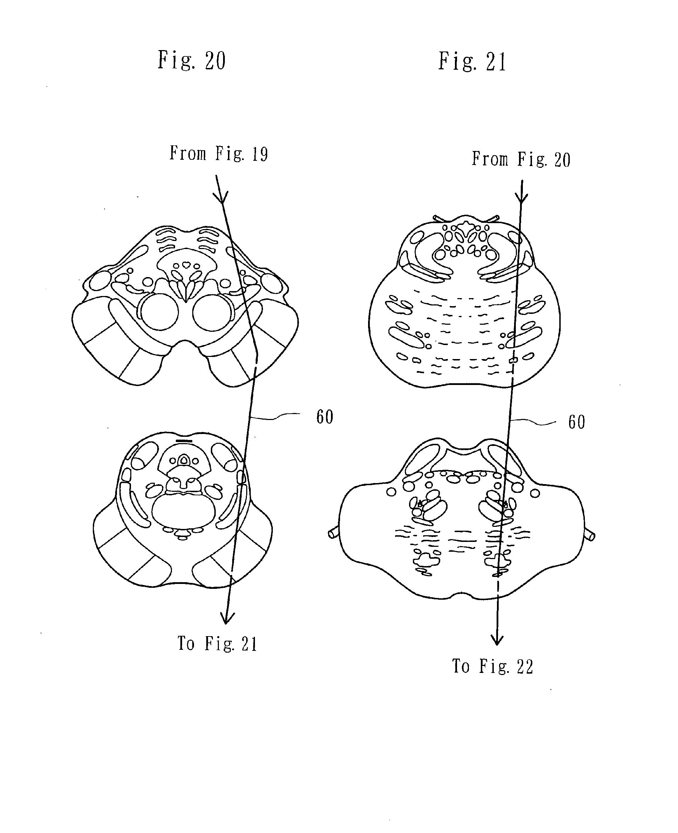

Figs. 19 through 25 are a series of nerve pathway cut surface diagrams which are displayed together on the display unit in the case when the motor nerve pathway display button 53 is pressed to select a motor nerve pathway in the nerve pathway selection data input screen page of Fig. 18.

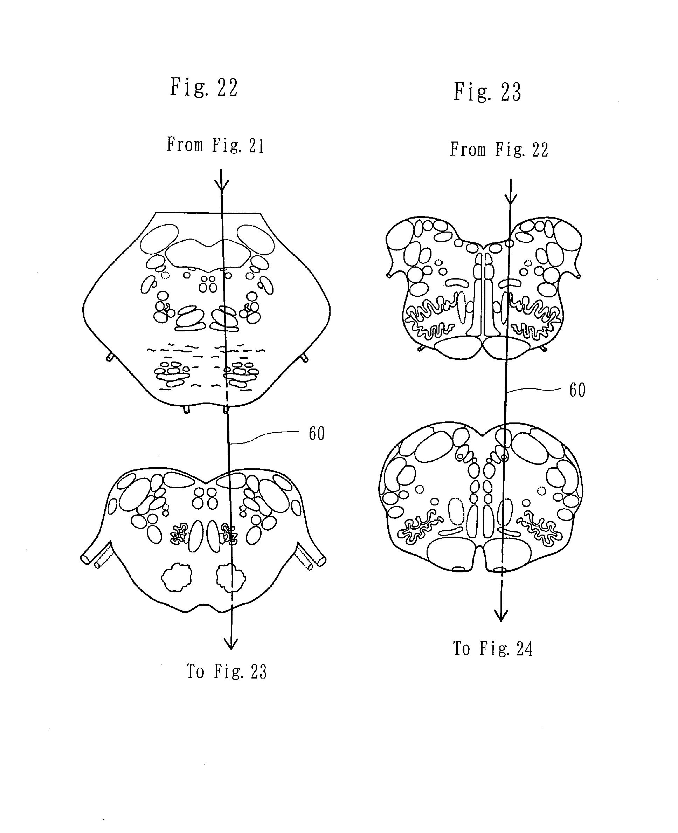

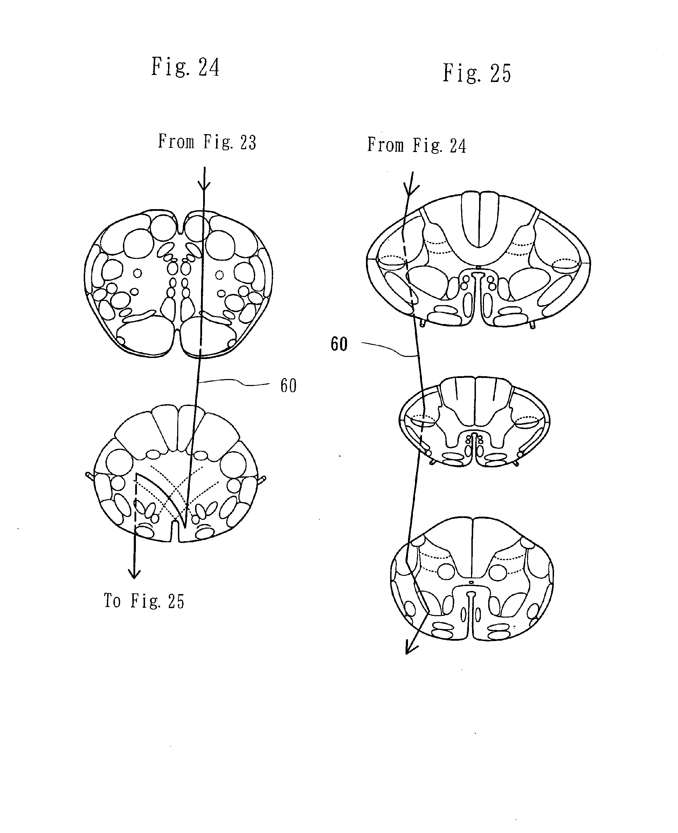

Fig. 19 shows a cerebrum coronary cut surface, and Fig. 20 shows a mesencephalon upper part cut surface following to the underside of the cerebrum coronary cut surface of Fig. 19, and a mesencephalon lower part cut surface following to the underside thereof. Fig. 21 shows a pons upper part cut surface following to the underside of the mesencephalon lower part cut surface of Fig. 20, and a pons middle part cut surface following to the underside thereof, and Fig. 22 shows a pons lower part cut surface following to the underside of the pons middle part cut surface of Fig. 21, and a medulla oblongata upper part cut surface following to the underside thereof. Fig. 23 shows a medulla oblongata upper-middle part cut surface following to the underside of the medulla oblongata upper part cut surface of Fig. 22, and a medulla oblongata middle part cut surface following to the underside thereof, and Fig. 24 shows a medulla oblongata middle-lower part cut surface following to the underside of the medulla oblongata middle part cut surface of Fig. 23, and a medulla oblongata lower part cut surface following to the underside thereof. Fig. 25 shows a cervical segment cut surface following to the medulla oblongata lower part cut surface of Fig. 24, a thoracic segment cut surface following to the underside thereof, and a lumbar segment cut surface following to the underside thereof.

As shown in Figs. 19 through 25, the motor nerve pathway 20 is displayed in a series of nerve pathway cut surface.

Furthermore, the system of the present invention is provided with a nerve pathway cut surface selection data input unit 46 for receiving selection data input for a nerve pathway cut surface which is intended to individually display among the nerve pathway cut surfaces displayed on the display unit 41 by means of the nerve pathway cut surface indication unit 42, an individual nerve pathway cut surface data extraction unit 47 for extracting data for drawing a relevant nerve pathway cut surface from the data stored in the nerve pathway cut surface data recording unit 40 based on the data received by the nerve pathway cut surface selection data input unit 46, an individual nerve pathway cut surface indication unit 48 for displaying a relevant nerve pathway cut surface on the display unit 41 based on the data extracted by the individual nerve pathway cut surface data extraction unit 47, and a nerve pathway-nerve nucleus name indication unit 49 for displaying a name of a nerve pathway or a nerve nucleus which is selected in the nerve pathway cut surface displayed on the display unit 41 by means of the individual nerve pathway cut surface indication unit 48.

When, for instance, a series of nerve pathway cut surfaces are displayed on the display unit 41 and any of the cut surfaces is double-clicked by means of a pointing device such as a mouse, selection data for the cut surface selected is input to the nerve pathway cut surface selection data input unit 46.

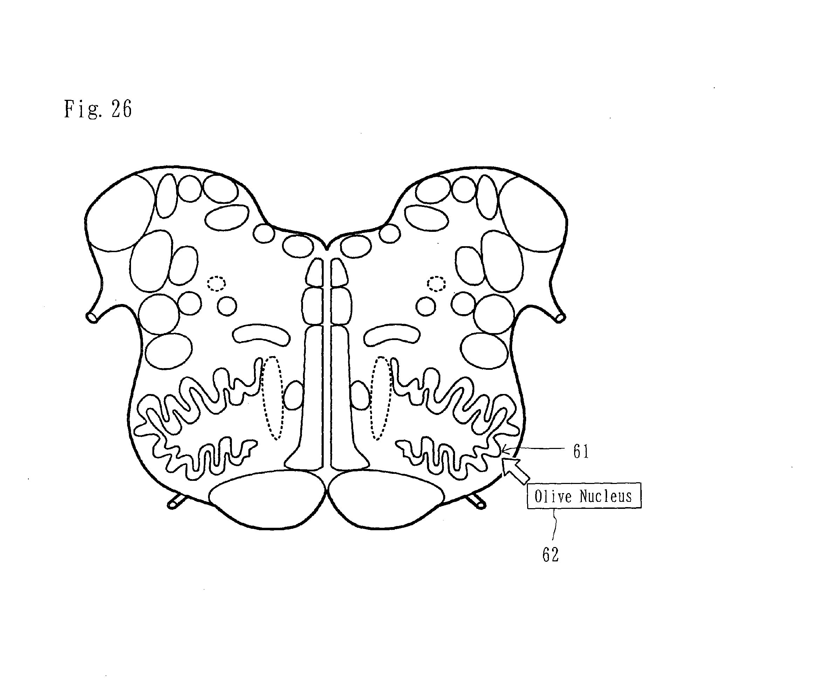

Now, when the medulla oblongata upper-middle part cut surface on the upper side in Fig. 23 is selected, the medulla oblongata upper-middle part cut surface is displayed in an enlarged manner on the display unit 41 as shown in Fig. 26 by means of the individual nerve pathway cut surface indication unit 48. On the page screen shown in Fig. 26, when, for embodiment, a position of a region 61 is specified by a pointing device such as a mouse, a name of a corresponding nerve pathway or name of nerve nucleus is displayed in a window 62, and in this figure, a name of nerve nucleus "olive nucleus" is displayed in the window 62 by means of the nerve pathway-nerve nucleus name indication unit 49.

### INDUSTRIAL APPLICABILITY

According to the present invention, when a medical physician inputs only data of a normal finding or an abnormal finding to a topical nerve diagnostic system in every neural finding items, relevant responsible nerve pathways are automatically displayed on a display of a computer together with a whole nerve pathway diagram, so that a responsible lesion with respect to a neural disorder of a patient is automatically displayed. Thus, the medical physician can give a rapid and correct diagnosis in case of topical nerve diagnosis without relying upon own experience and gut feeling unlike in a conventional manner. Therefore, the present invention contributes remarkably to medical equipment affiliated industries as a kind of diagnosis support system in a topical nerve diagnosis.

Moreover, according to the present invention, a medical student can learn visually positions and mutual physical relationships in a whole nerve pathway diagram of respective nerve pathways in human body, besides positions and names of nerve pathways and nerve nuclei belonging to nerve pathway cut surfaces in every cut surfaces thereof by observing cut surfaces and nerve pathways in a specified region in the whole nerve pathway diagrams displayed sequentially on a display of a computer as well as watching individual enlarged diagrams of the cut surfaces displayed on the display. As a result, the medical student can understand easily and memorize efficiently nerve pathway diagrams and nerve cut surfaces in neuroanatomy. Therefore, the present invention contributes significantly to medical educational material affiliated industries as an assisting means for medical education relating to neuroanatomy.

## Claims

1. A topical nerve diagnostic system with the use of a computer, **characterized by** that said topical nerve diagnostic system comprises:
a whole nerve pathway diagram data recording unit for storing data of whole nerve pathway diagrams;
a nerve finding data input unit for receiving normal finding or abnormal finding data input with respect to respective neural finding items;
a responsible nerve pathway data extraction unit for extracting data for drawing a responsible nerve pathway relating to neural finding items being in an abnormal finding from the data stored in said whole nerve pathway diagram data recording unit based on the data received in said nerve finding data input unit;
a display unit;
a whole nerve pathway indication unit for displaying a whole nerve pathway diagram on said display unit based on the data stored in said whole nerve pathway diagram data recording unit;
a responsible nerve pathway indication unit for displaying a responsible nerve pathway in the whole nerve pathway diagram displayed on said display unit by said whole nerve pathway indication unit based on the data extracted by said responsible nerve pathway data extraction unit; and
a responsible lesion estimation/indication unit for presuming a position of a responsible lesion in said whole nerve pathway diagram based on the responsible nerve pathway displayed on said display unit by said responsible nerve pathway indication unit.

2. The topical nerve diagnostic system according to claim 1, **characterized by** that the data stored in said whole nerve pathway diagram data recording unit contains data of at least names and positions of respective nerve nuclei in the whole nerve pathway diagram, connection relations in the respective nerve nuclei, and curves or straight lines representing nerve fascicles for connecting the nerve nuclei with each other.

3. The topical nerve diagnostic system according to claim 2, **characterized by** that said responsible nerve pathway data extraction unit is adapted to extract data of relevant names and positions of nerve nuclei in the whole nerve pathway diagram, relevant connection relations in the respective nerve nuclei, and curves or straight lines representing nerve fascicles for connecting the relevant nerve nuclei with each other from said whole nerve pathway diagram data recording unit in every neural finding items exhibiting abnormal findings.

4. The topical nerve diagnostic system according to claim 3, **characterized by** that said responsible lesion estimation/indication unit is adapted to detect a region where responsible nerve pathways displayed on said display unit intersect with each other and a region where said responsible nerve pathways approach one another in the closest relation, and presume the region detected to be a responsible lesion thereby to display the responsible lesion in said whole nerve pathway diagram on said display unit.

5. The topical nerve diagnostic system according to claim 4, **characterized by** that said topical nerve diagnostic system further comprises:
a nerve pathway cut surface data recording unit for storing cut surface data in a specified region in said whole nerve pathway diagram;
a cut surface display region selection data input unit for receiving selection data input of a specified region in which a cut surface is to be displayed in the whole nerve pathway diagram displayed on said display unit;
a second responsible nerve pathway data extraction unit for extracting data for drawing a responsible nerve pathway relating to a neural finding item to be in an abnormal finding onto a cut surface of relevant specified region from the data stored in said nerve pathway cut surface data recording unit based on the data received by said cut surface display region selection data input unit and the data received by said nerve finding data input unit;
a nerve pathway cut surface indication unit for extracting relevant cut surface data from the data stored in said nerve pathway cut surface data recording unit based on the data received by said cut surface display region selection data input unit thereby to display said relevant cut surface;
a second responsible nerve pathway indication unit for displaying a responsible nerve pathway in the nerve pathway cut surface displayed by said nerve pathway cut surface indication unit based on the data extracted by said second responsible nerve pathway data extraction unit; and
a second responsible lesion estimation/indication unit for presuming a position of a responsible lesion in said relevant cut surface based on the responsible nerve pathway displayed on said display unit by said second responsible nerve pathway indication unit thereby to display the responsible lesion presumed in said relevant cut surface.

6. The topical nerve diagnostic system according to claim 5, **characterized by** that the data stored in said nerve pathway cut surface data recording unit contains data of relevant respective names and positions of nerve nuclei in the cut surface, connection relations in relevant respective nerve nuclei, and curves or straight lines representing nerve fascicles for connecting the relevant nerve nuclei with each other in said every cut surfaces.

7. The topical nerve diagnostic system according to claim 6, **characterized by** that said second responsible nerve pathway data extraction unit is adapted to extract data of relevant respective names and positions of nerve nuclei in the cut surface, connection relations in relevant respective nerve nuclei, and curves or straight lines representing nerve fascicles for connecting the relevant nerve nuclei with each other from the relevant cut surface data stored in said nerve pathway cut surface data recording unit in every neural finding items to be in abnormal findings.

8. The topical nerve diagnostic system according to claim 5, **characterized by** that said second responsible lesion estimation/indication unit is adapted to detect a region where responsible nerve pathways displayed on said display unit intersect with each other and a region where said responsible nerve pathways approach one another in the closest relation, and presume the region detected to be a responsible lesion thereby to display the responsible lesion presumed in said cut surface.

9. The topical nerve diagnostic system according to claim 5, **characterized by** having a screen page switchover unit for switching over a screen page of said whole nerve pathway diagram in said display unit to a screen page of a cut surface in a specified region of said whole nerve pathway diagram.

10. The topical nerve diagnostic system according to any one of claims 1 to 9, **characterized by** that said neural finding items include oculomotor restriction, inferior oculomotor restriction, jaw reflex acceleration, impaired facial tactual sensation, impaired facial pain/temperature sensation, corneal areflexia, exterior oculomotor restriction no, upper facial paralysis, lower facial paralysis, impaired taste, lowered pharyngeal reflex/swallowing difficulty, impaired pharyngeal sound dysphemia, lingual muscle paralysis/impaired lingual sound dysphemia, sternocleidomastoid paralysis, impaired upper limb pain/temperature sensation, impaired upper limb deep sensation, upper limb motor paralysis, superior limb tendon reflex, impaired trunk pain/temperature sensation, impaired trunk deep sensation, level of impaired trunk deep sensation, impaired lower limb pain/temperature sensation, inferior bathyesthesia disorder, lower limb motor paralysis, inferior limb tendon reflex, and Babinski reflex.

11. The topical nerve diagnostic system according to claim 1, **characterized by** that the data stored in said whole nerve pathway diagram data recording unit contains at least data of names and positions of respective spinal roots, respective muscles and respective skin areas in the whole nerve pathway diagram, connection relations in the respective spinal roots and the respective muscles, and curves or straight lines representing nerve fascicles for connecting the respective spinal roots with the respective skin as well as data of connection relations in the respective spinal roots and the respective skin areas, and curves or straight lines for connecting the respective spinal roots with the respective skin areas.

12. The topical nerve diagnostic system according to claim 11, **characterized by** that said responsible nerve pathway data extraction unit is adapted to extract data of relevant names and positions of spinal roots, muscles and skin areas in the whole nerve pathway diagram, relevant connection relations in the respective spinal roots and the respective muscles, and curves or straight lines representing nerve fascicles for connecting the relevant respective spinal roots with the respective skins as well as data of relevant connection relations in the respective spinal roots and the respective skin areas, and curves or straight lines for connecting the relevant respective spinal roots with the respective skin areas from said whole nerve pathway diagram data recording unit in every neural finding items which are to be in abnormal findings.

13. The topical nerve diagnostic system according to claim 12, **characterized by** that said responsible lesion estimation/indication unit is adapted to detect a region where responsible nerve pathways displayed on said display unit overlap each other at the highest degree, and presume the region detected to be a responsible lesion thereby to display the responsible lesion presumed in said whole nerve pathway diagram on said display unit.

14. The topical nerve diagnostic system according to claim 13, **characterized by** having further a third responsible lesion estimation/indication unit excluding a responsible nerve pathway part corresponding to nerve fascicles for connecting a muscle or skin region in which finding data input comes to be a normal finding with the spinal roots relating thereto from the responsible nerve pathways displayed in said whole nerve pathway diagram on the display unit by means of said responsible lesion estimation/indication unit in the case when the finding data input as to abnormality of respective muscles or respective skin regions relating to said responsible nerve pathways is received by said nerve finding data input unit.

15. The topical nerve diagnostic system according to claim 14, **characterized by** that said neural finding items include perception disorder in respective muscle and skin regions relating to movements of respective articulations.

16. A neuroanatomy learning system with the use of a computer, **characterized by** that said neuroanatomy learning system comprises:
a nerve pathway cut surface data recording unit for recording cut surface data in at least one region of cerebrum and mesencephalon, at least one region of pons, at least one region of medulla oblongata, and at least one region of spinal cord, respectively, in a whole pathway diagram;
a display unit;
a nerve pathway cut surface indication unit for displaying cut surfaces of at least one region of the cerebrum and the mesencephalon, at least one region of the pons, at least one region of the medulla oblongata, at least one region of the medulla oblongata, and at least one region of the spinal cord, respectively, in this order based on the data stored in said nerve pathway cut surface data recording unit;
a nerve pathway selection data input unit for receiving selection data input of nerve pathways to be displayed on said display unit;
a nerve pathway data extraction unit for extracting data for drawing relevant nerve pathways from the data stored in said nerve pathway cut surface data recording unit based on the data received by the nerve pathway selection data input unit in every nerve pathway cut surfaces;
a nerve pathway indication unit for displaying relevant nerve pathways in a nerve pathway cut surface displayed by said nerve pathway cut surface indication unit based on the data extracted by said nerve pathway data extraction unit;
a nerve pathway cut surface selection data input unit for receiving selection data input for a nerve pathway cut surface which is intended to individually display among the nerve pathway cut surfaces displayed on said display unit by means of said nerve pathway cut surface indication unit;
an individual nerve pathway cut surface data extraction unit for extracting data for drawing a relevant nerve pathway cut surface from the data stored in said nerve pathway cut surface data recording unit based on the data received by said nerve pathway cut surface selection data input unit;
an individual nerve pathway cut surface indication unit for displaying a relevant nerve pathway cut surface on said display unit based on the data extracted by said individual nerve pathway cut surface data extraction unit; and
a nerve pathway-nerve nucleus name indication unit for displaying a name of a nerve pathway or a nerve nucleus which is selected in the nerve pathway cut surface displayed on said display unit by means of said individual nerve pathway cut surface indication unit.

17. The neuroanatomy learning system according to claim 16, **characterized by** that the data stored in said nerve pathway cut surface data recording unit contains data of relevant names and positions of nerve nuclei in said cut surfaces, relevant connection relations in the nerve nuclei, and curves or straight lines representing nerve fascicles for connecting relevant nerve nuclei with each other, and names of relevant nerve pathway and positions in said cut surfaces in every cut surfaces.

18. The neuroanatomy learning system according to claim 16 or 17, **characterized by** that at least one region of said mesencephalon consists of the upper part of the mesencephalon and the lower part of the mesencephalon, at least one region of said pons consists of the upper, the middle, and the lower parts of the pons, at least one region of the medulla oblongata consists of the upper part, the upper-middle part, the middle, the middle-lower part, and the lower part of the medulla oblongata, and at least one region of said spinal cord consists of a cervical segment, a thoracic segment, and a lumbar segment.
